# NEW EUROPEAN PATENT SPECIFICATION

(11) **EP 2 651 958 B2**
(45) Date of publication and mention of the opposition decision: **30.11.2022**
(45) Mention of the grant of the patent: 03.07.2019
(21) Application number: 11848181.1
(22) Date of filing: 13.12.2011
(51) Int. Cl.: C07H 15/207, A23L 2/60, A24B 15/40, A61K 8/60, A61Q 11/00, C07H 15/24, A23L 27/10, A23L 27/30

(54) **GLYCOSIDE BLENDS**
GLYKOSIDMISCHUNGEN
MÉLANGES DE GLYCOSIDES

(30) Priority: 13.12.2010 US 422523 P
(43) Date of publication of application: 23.10.2013
(60) Divisional application: 19162164.8
(73) Proprietor: Cargill, Incorporated, Wayzata, MN 55391 (US)
(72) Inventor: CARLSON, Ting Liu, Dayton, Ohio 45458 (US); GUTHRIE, Brian D., Chanhassen, Minnesota 55317 (US); LINDGREN, Timothy, Crystal, Minnesota 55427 (US); MORTENSON, Michael, Rogers, MN 55374 (US)
(74) Representative: Forresters IP LLP
(86) International application number: PCT/US2011/064528
(87) International publication number: WO 2012/082677

(56) References cited:
- EP-A2- 0 154 235
- WO-A1-2008/112872
- WO-A1-2011/090709
- WO-A1-2012/102769
- WO-A2-2008/091547
- JP-A- S5 283 731
- JP-A- 2007 195 449
- US-A1- 2007 082 106
- US-A1- 2007 116 823
- US-A1- 2007 116 823
- US-A1- 2008 107 775
- US-A1- 2010 015 320
- US-A1- 2010 099 857
- CHATSUDTHIPONG ET AL.: 'Stevioside and related compounds: Therapeutic benefits beyond sweetness' PHARMACOLOGY & THERAPEUTICS vol. 121, no. 1, 2009, pages 41 - 54, XP025816758
- CHATSUDTHIPONG V ET AL: "Stevioside and related compounds: Therapeutic benefits beyond sweetness", PHARMACOLOGY AND THERAPEUTICS, ELSEVIER, GB, vol. 121, no. 1, 1 January 2009 (2009-01-01), pages 41-54, XP025816758, ISSN: 0163-7258, DOI: 10.1016/J.PHARMTHERA.2008.09.007 [retrieved on 2008-10-27]

## Description

### FIELD OF THE INVENTION

The present invention relates to sweetener compositions comprising glycoside blends. The sweetener compositions of the present invention can further comprise other ingredients. The sweetener compositions of the present invention further comprise one or more bulking agents. The present invention also relates to incorporation of the sweetener compositions into foods and/or beverages.

### BACKGROUND OF THE INVENTION

The species *Stevia rebaudiana ("Stevia")* has been the subject of considerable research and development efforts directed at the purification of certain naturally occurring sweet glycosides of *Stevia* that have potential as non-caloric sweeteners. Sweet glycosides that may be extracted from *Stevia* include the six rebaudiosides (i.e., rebaudioside A to F), stevioside, and dulcoside A. In particular, significant commercial interest has been focused on obtaining and purifying rebaudioside A from *Stevia.* <WO 2008/091547, US 2007/116823 and US 2010/015320 relate to sweetner compositions. WO 2008/112872 and US 2007/082106 relate to beverages. JP 2007 195449 relates to beverages and food stuffs.>

### SUMMARY OF THE INVENTION

The invention is defined in the claims.

The present invention relates to sweetener compositions having particular glycoside blends. The sweetener composition of the present invention further comprises a bulking agent. The sweetener compositions of the present invention can also include other ingredients such as flavorings, other high intensity sweeteners, or the like. The present invention also pertains to the use of the sweetener compositions in foods and beverages.

Applicants have surprisingly discovered that certain blends of rebaudioside A and rebaudioside B, in binary and ternary forms, result in blends which have higher effective sweetening ability than the pure component steviol glycosides of which the blends are made. That is, the same level of sweetness can be achieved with a lower concentration of the blend of glycosides than the amount that would be needed with the pure component rebaudioside A or rebaudioside B, component. The reduction in concentration of glycoside needed to achieve a certain level of sweetness can result in ample savings by allowing the utilization of lower amounts of the glycoside in sweetener compositions yet achieving the same level of sweetness. Additionally, lower levels of glycoside could allow for easier incorporation into certain foods and beverages. In some embodiments, the added benefit of reduced bitterness (while attaining the same sweetness) is also achieved.

In certain preferred embodiments, the blends are high purity glycoside blends. In other preferred embodiments, the glycoside blends provide relatively high sucrose equivalent value ("SEV") in the sweetener compositions. In these embodiments, when a higher level of sweetness is needed in sweetener compositions for certain food or beverage applications, the substantial benefit that the glycoside blends provide could better be realized.

The invention features a sweetener composition comprising a glycoside blend. The glycoside blend comprises from 40% to 85% rebaudioside A and from 15% to 60% rebaudioside B (of the total rebaudioside A and rebaudioside B in the glycoside blend), and the glycoside blend provides an SEV of greater than 7.2 in the sweetener composition, and rebaudioside A and rebaudioside B comprise at least 60% of the glycoside blend.

An aspect of the disclosure features a sweetener composition comprising a glycoside blend. The glycoside blend comprises from 10% to 55% rebaudioside A, from 30% to 75% rebaudioside B, and 10% to 30% rebaudioside D (of the total rebaudioside A, rebaudioside B, and rebaudioside D in the glycoside blend), and the glycoside blend provides an SEV of greater than 3.9 in the sweetener composition, and rebaudioside A, rebaudioside B, and rebaudioside D comprise at least 70% of the glycoside blend.

Other objects, features, and advantages of the invention will be apparent from the following detailed description and claims.

### BRIEF DESCRIPTION OF THE DRAWINGS

Figure 1 is a table showing the sweet and bitter response of rebaudioside B and rebaudioside D blends (REB-BD glycoside blends).
Figure 2 is a table showing sweet and bitter response of rebaudioside A and rebaudioside D blends (REB-AD glycoside blends).
Figure 3 is a table showing sweet and bitter response of rebaudioside A and rebaudioside B blends (REB-AB glycoside blends).
Figure 4 is a table showing sweet and bitter response of rebaudioside A, rebaudioside B, and rebaudioside D blends (REB-ABD glycoside blends).

### DETAILED DESCRIPTION OF THE INVENTION

### Introduction

The term "glycoside blend" as used herein means a blend of the various glycosides obtained from the Stevia plant. These glycosides include, but are not limited to, rebaudiosides A-F, stevioside, dulcoside, steviobioside, and rubusoside. In particular, the glycoside blends of the present invention include blends consisting predominantly of rebaudioside A and rebaudioside B.

The term "REB-AD glycoside blend", as used herein, refers to a glycoside blend in which the primary components of the glycoside blend are rebaudioside A and rebaudioside D. In a REB-AD glycoside blend, the combination of rebaudioside A and rebaudioside D will make up at least 60% of the total glycosides in the glycoside blend.

The term "REB-AB glycoside blend", as used herein, refers to a glycoside blend in which the primary components of the glycoside blend are rebaudioside A and rebaudioside B. In a REB-AB glycoside blend, the combination of rebaudioside A and rebaudioside B will make up at least 60% of the total glycosides in the glycoside blend.

The term "REB-BD glycoside blend", as used herein, refers to a glycoside blend in which rebaudioside B and rebaudioside D make up a significant portion of the glycoside blend. In a REB-BD glycoside blend, the combination of rebaudioside B and rebaudioside D will make up at least 30% of the total glycosides in the glycoside blend.

The term "REB-ABD glycoside blend", as used herein, refers to a glycoside blend in which the primary components of the glycoside blend are rebaudioside A, rebaudioside B, and rebaudioside D. In a REB-ABD glycoside blend, the combination of rebaudioside A, rebaudioside B, and rebaudioside D will make up at least 70% of the total glycosides in the glycoside blend.

Rebaudioside A is a compound having the following chemical structure:

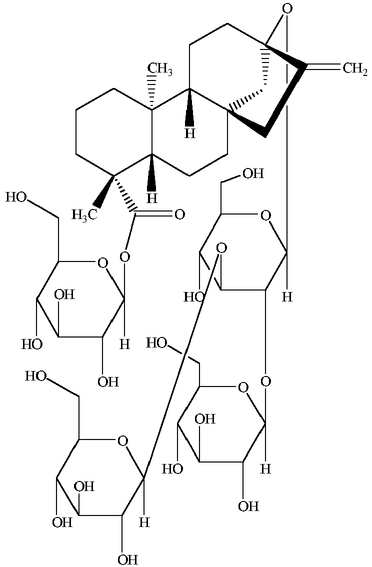

Rebaudioside B is a compound having the following chemical structure:

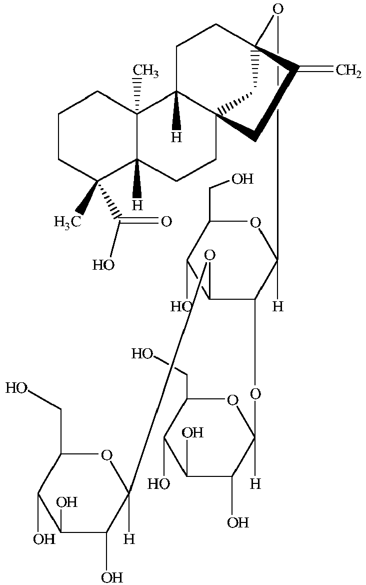

Rebaudioside D is a compound having the following chemical structure:

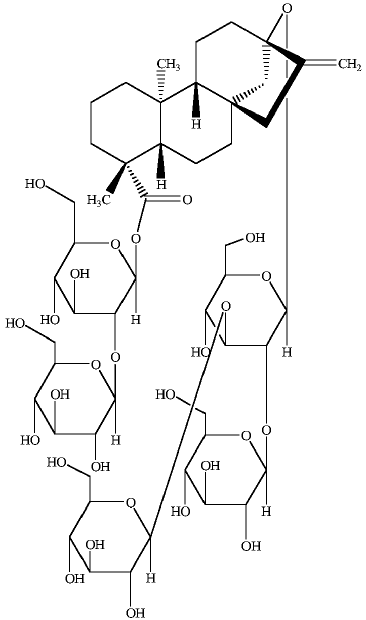

### Sweetener Compositions with Glycoside Blends

### REB-AB Glycoside Blends

Applicants have surprisingly discovered that at certain SEV levels, certain blends of rebaudioside A and rebaudioside B surprisingly have higher sweetening ability than either pure rebaudioside A or pure rebaudioside B.

In some embodiments, the sweetener compositions include a REB-AB glycoside blend wherein the REB-AB glycoside blend comprises from 40% to 85% rebaudioside A and from 15% to 60% rebaudioside B (of the total rebaudioside A and rebaudioside B in the glycoside blend), and wherein the REB-AB glycoside blend provides an SEV of greater than 3.6 in the sweetener composition. In other embodiments, the sweetener compositions include a REB-AB glycoside blend wherein the REB-AB glycoside blend comprises from 42% to 82% rebaudioside A and from 18% to 58% rebaudioside B (of the total rebaudioside A and rebaudioside B in the glycoside blend), and wherein the REB-AB glycoside blend provides an SEV of greater than 7.2 in the sweetener composition.

The disclosed REB-AB glycoside blends provides an SEV of greater than 4.0, 5.0, 6.5, or 7.2 in the sweetener composition. In yet other embodiments, the REB-AB glycoside blend provides an SEV that ranges from 3.7 to 9.0 to the sweetener composition. In yet other embodiments, the REB-AB glycoside blend provides an SEV that ranges from 6.0 to 8.5 to the sweetener composition. In yet other embodiments, the REB-AB glycoside blend provides an SEV that ranges from 7.3 to 8.0 to the sweetener composition.

The combination of rebaudioside A and rebaudioside B in REB-AB glycoside blends will make up considerable percentage of the total of all glycosides in the blends. The remaining portion of these REB-AB glycoside blends can be made up of various concentrations of the remaining glycosides which may be obtained from the Stevia plant (rebaudiosides C, D, E, and F, stevioside, dulcoside, rubusoside, etc).

In some embodiments, the combination of rebaudioside A and rebaudioside B makes up at least 60% of the REB-AB glycoside blend. In other embodiments, the combination of rebaudioside A and rebaudioside B makes up at least 70% of the REB-AB glycoside blend. In yet other embodiments, the combination of rebaudioside A and rebaudioside B makes up at least 80% of the REB-AB glycoside blend. In yet other embodiments, the combination of rebaudioside A and rebaudioside B makes up at least 90% of the REB-AB glycoside blend.

In some particular embodiments, it may be desired that rebaudioside A and rebaudioside B make up even more of the total REB-AB glycoside blend. In some of these embodiments, the combination of rebaudioside A and rebaudioside B makes up at least 93% of the REB-AB glycoside blend. In other embodiments, the combination of rebaudioside A and rebaudioside B makes up at least 95% of the REB-AB glycoside blend. In yet other embodiments, the combination of rebaudioside A and rebaudioside B makes up at least 97% of the REB-AB glycoside blend. In yet other embodiments, the combination of rebaudioside A and rebaudioside B makes up at least 98% of the REB-AB glycoside blend.

All of the sweetener compositions with REB-AB glycoside blends at the rebaudioside A and rebaudioside B ratios and SEV values disclosed herein are also contemplated at the purity levels described herein.

Without being bound by theory, applicants believe that, at particular SEV levels and ratios, a higher level of purity in the REB-AB blend could allow for improved sweetness synergism between rebaudioside A and rebaudioside B without substantial hindrance from the other glycosides.

In some particularly preferred embodiments, the sweetener compositions include a REB-AB glycoside blend wherein the REB-AB glycoside blend comprises from 40% to 85% rebaudioside A and from 15% to 60% rebaudioside B (of the total rebaudioside A and rebaudioside B in the glycoside blend), wherein the REB-AB glycoside blend provides an SEV of greater than 7.0 in the sweetener composition, and wherein the combination of rebaudioside A and rebaudioside B makes up at least 80% of the REB-AB glycoside blend. In other particularly preferred embodiments, the sweetener compositions include a REB-AB glycoside blend wherein the REB-AB glycoside blend comprises from 42% to 82% rebaudioside A and from 18% to 58% rebaudioside B (of the total rebaudioside B and rebaudioside D in the glycoside blend), wherein the REB-AB glycoside blend provides an SEV of greater than 7.2 in the sweetener composition, and wherein the combination of rebaudioside A and rebaudioside B makes up at least 90% of the REB-AB glycoside blend.

### REB-ABD Glycoside Blends

Certain ternary blends of rebaudioside A, rebaudioside B, and rebaudioside D, at certain SEV levels, were surprisingly found to have improved sweetening ability compared to pure rebaudioside A, rebaudioside B, or rebaudioside D.

In some embodiments, the sweetener compositions include a REB-ABD glycoside blend wherein the REB-ABD glycoside blend comprises from 10% to 55% rebaudioside A, from 30% to 75% rebaudioside B, and from 10% to 30% rebaudioside D (of the total rebaudioside A, rebaudioside B, and rebaudioside D in the glycoside blend), and wherein the REB-ABD glycoside blend provides an SEV of greater than 3.9 in the sweetener composition. In other embodiments, the sweetener compositions include a REB-ABD glycoside blend wherein the REB-ABD glycoside blend comprises from 15% to 52% rebaudioside A, from 32% to 71% rebaudioside B, and from 14% to 25% rebaudioside D (of the total rebaudioside A, rebaudioside B, and rebaudioside D in the glycoside blend), and wherein the REB-ABD glycoside blend provides an SEV of greater than 3.9 in the sweetener composition.

In other embodiments, the REB-ABD glycoside blend provides an SEV of greater than 5.0, 6.0, 7.0, or 7.2 in the sweetener composition. In yet other embodiments, the REB-ABD glycoside blend provides an SEV that ranges from 6.0 to 9.0 to the sweetener composition. In yet other embodiments, the REB-ABD glycoside blend provides an SEV that ranges from 7.0 to 8.5 to the sweetener composition. In yet other embodiments, the REB-ABD glycoside blend provides an SEV that ranges from 7.6 to 8.0 to the sweetener composition.

The combination of rebaudioside A, rebaudioside B, and rebaudioside D in REB-ABD glycoside blends will make up considerable percentage of the total of all glycosides in the blends. The remaining portion of these REB-ABD glycoside blends can be made up of various concentrations of the remaining glycosides which may be obtained from the Stevia plant (rebaudiosides C, E, and F, stevioside, dulcoside, etc).

In some embodiments, the combination of rebaudioside A, rebaudioside B, and rebaudioside D makes up at least 70% of the REB-ABD glycoside blend. In other embodiments, the combination of rebaudioside A, rebaudioside B, and rebaudioside D makes up at least 80% of the REB-ABD glycoside blend. In yet other embodiments, the combination of rebaudioside A, rebaudioside B, and rebaudioside D makes up at least 90% of the REB-ABD glycoside blend.

In some particular embodiments, it may be desired that rebaudioside A, rebaudioside B, and rebaudioside D make up even more of the total REB-ABD glycoside blend. In some of these embodiments, the combination of rebaudioside A, rebaudioside B, and rebaudioside D makes up at least 93% of the REB-ABD glycoside blend. In other embodiments, the combination of rebaudioside A, rebaudioside B, and rebaudioside D makes up at least 95% of the REB-ABD glycoside blend. In yet other embodiments, the combination of rebaudioside A, rebaudioside B, and rebaudioside D makes up at least 97% of the REB-ABD glycoside blend. In yet other embodiments, the combination of rebaudioside A, rebaudioside B, and rebaudioside D makes up at least 98% of the REB-ABD glycoside blend.

In some particularly preferred embodiments, the sweetener compositions include a REB-ABD glycoside blend wherein the REB-ABD glycoside blend comprises from 10% to 55% rebaudioside A, from 30% to 75% rebaudioside B, and from 10% to 30% rebaudioside D (of the total rebaudioside A, rebaudioside B, and rebaudioside D in the glycoside blend), and wherein the REB-ABD glycoside blend provides an SEV of greater than 6.0 in the sweetener composition, and wherein the combination of rebaudioside A, rebaudioside B, and rebaudioside D makes up at least 85% of the REB-ABD glycoside blend. In other particularly preferred embodiments, the sweetener compositions include a REB-ABD glycoside blend wherein the REB-ABD glycoside blend comprises from 15% to 52% rebaudioside A, from 32% to 71% rebaudioside B, and from 14% to 25% rebaudioside D (of the total rebaudioside A, rebaudioside B, and rebaudioside D in the glycoside blend), and wherein the REB-ABD glycoside blend provides an SEV of greater than 7.2 in the sweetener composition, and wherein the combination of rebaudioside A, rebaudioside B, and rebaudioside D makes up at least 90% of the REB-AB glycoside blend.

### Other Ingredients of the Sweetener Compositions

The sweetener compositions of the present inventions including a particular glycoside blend can also include other ingredients. The sweetener composition of the present invention further comprises a bulking agent. In some embodiments, the sweetener composition can further comprise one or more of a high-intensity sweetener, a flavoring, an antioxidant, caffeine, other nutritive sweetener, salts, protein, or a sweetness enhancer.

A bulking agent can include any compositions known in the art used to add bulk to high intensity sweeteners. A bulking agent may be chosen from a bulk sweetener, a lower glycemic carbohydrate, a fiber, a hydrocolloid, and combinations thereof. A bulk sweetener may be chosen from corn sweeteners, sucrose, dextrose, invert sugar, maltose, dextrin, maltodextrin, fructose, levulose, high fructose corn syrup, corn syrup solids, galactose, trehalose, isomaltulose, fructo-oligosaccharides, and combinations thereof. A lower glycemic carbohydrate may be chosen from fructo-oligosaccharide, galactooligosaccharide, isomaltooligosaccharide, oligodextran, D-tagatose, sorbitol, mannitol, xylitol, lactitol, erythritol, maltitol, other polyols, hydrogenated starch hydrolysates, isomalt, D-psicose, 1,5 anhydro D-fructose, and combinations thereof.

A fiber may be chosen from polydextrose, resistant maltodextrin, resistant starch, inulin, soluble corn fiber, beta-glucan, psyllium, cellulose, hemicellulose, and combinations thereof. A hydrocolloid may be chosen from pectin (apple, beet, citrus), gum Arabic, guar gum, carboxymethylcellulose, nOSA (n-octenyl succinic anhydride), locust bean gum, cassia gum, xanthan gum, carrageenan, alginate, and combinations thereof.

A high intensity sweetener may be chosen from sucralose, aspartame, saccharin, acesulfame K, alitame, thaumatin, dihydrochalcones, neotame, cyclamates, mogroside, glycyrrhizin, phyllodulcin, monellin, mabinlin, brazzein, circulin, pentadin, and combinations thereof. A flavoring may be chosen from a cola flavor, a citrus flavor, a root beer flavor, and combinations thereof. A sweetness enhancer may be chosen from curculin, miraculin, cynarin, chlorogenic acid, caffeic acid, strogins, arabinogalactan, maltol, dihyroxybenzoic acids, and combinations thereof.

Other ingredients such as food starch, flours, protein isolates, protein concentrates, food fats and oils (such as cocoa butter), food extracts (such as malt extract), and juice concentrates may also be included in the sweetener compositions.

In some particular embodiments, the sweetener composition comprising a glycoside blend can also include a lower glycemic carbohydrate. In certain preferred embodiments, the lower glycemic carbohydrate is erythritol or another polyol. In especially preferred embodiments, the sweetener composition includes a particular glycoside blend and erythritol.

In other particular embodiments, the sweetener composition comprising a glycoside blend can also include a fiber. In certain preferred embodiments the fiber is polydextrose, resistant maltodextrin, or inulin.

### Food and Beverage Compositions

The sweetener compositions of the present inventions can also be incorporated into food and beverage compositions. Thus, the present invention also contemplates food compositions and beverage compositions which include the sweetener compositions of the present invention.

### Methods of Producing Sweetener Compositions

The present invention also contemplates methods for producing the sweetener compositions. Typical conventional *Stevia* based sweeteners include a glycoside blend which consists primarily of rebaudioside A (for example greater than 95% rebaudioside A, or greater than 97% rebaudioside A).

The present invention contemplates adding rebaudioside B to such conventional sweeteners. In some embodiments, rebaudioside B could be added to such sweeteners to achieve the desired rebaudioside A to rebaudioside B glycoside blend ratio. In yet other embodiments of the disclosure, rebaudioside B and rebaudioside D could be added to such sweeteners to achieve the desired rebaudioside A to rebaudioside B to rebaudioside D glycoside blend ratio.

The present invention also contemplates controlled conversion between one glycoside and another glycoside to achieve the glycoside blends of the present invention. Thus, in one embodiment, a substantially pure rebaudioside A composition can be converted to particular REB-AB blend, REB-BD blend, or REB-ABD blend at the claimed ratios.

### REFERENCE EXAMPLE

### Example 1: Sensory Testing of Various Glycoside Blends

A 20 person sensory panel was trained to scale sweetness and bitterness. Reference tasting standards were prepared by dissolving respective standard material (sucrose for sweetness and caffeine for bitterness) into reverse osmosis water according to the scale values shown in Table 1 below.

**Table 1: Reference Tasting Standards**

| | Concentration (g/kg) | |
|---|---|---|
| Scale | Sucrose *(Sweetness)* | Caffeine *(Bitterness)* |
| 1 | 10 | 0.107 |
| 2 | 20 | 0.153 |
| 3 | 30 | 0.200 |
| 4 | 40 | 0.246 |
| 5 | 50 | 0.293 |
| 6 | 60 | 0.340 |
| 7 | 70 | 0.386 |
| 8 | 80 | 0.433 |
| 9 | 90 | 0.479 |
| 10 | 100 | 0.526 |
| 11 | 110 | 0.572 |
| 12 | 120 | 0.619 |
| 13 | 130 | 0.666 |
| 14 | 140 | 0.712 |
| 15 | 150 | 0.759 |

Pure rebaudioside A, rebaudioside B, and rebaudioside D were obtained. Rebaudioside A (99% purity) was obtained from ChromaDex^{®}. Rebaudioside B (97.3% purity) was obtained from Cargill, Incorporated. Rebaudioside D (92.5% purity) was obtained from a commercial source.

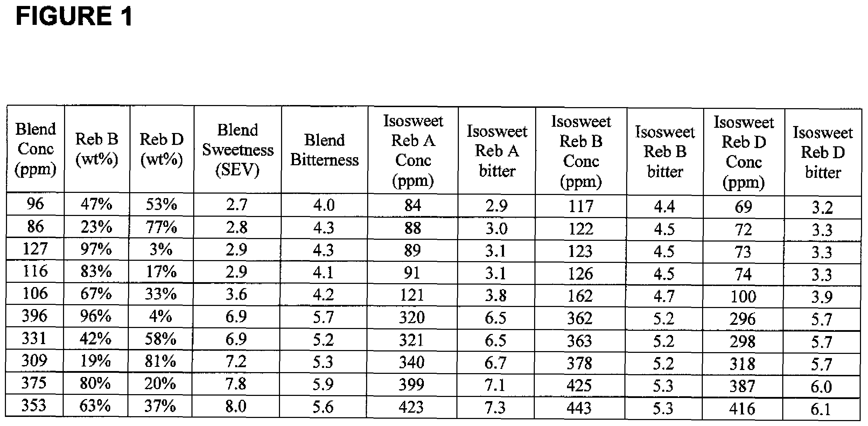

The trained sensory panel evaluated pure and blended solutions of rebaudioside A, rebaudioside B, and rebaudioside D at ratios and concentrations shown in the tables and in figures 1-4. Solutions were made in Evian^{®} water. All solutions were heated to 47°C for 10 minutes to ensure that all the glycoside material was completely dissolved. The solutions were allowed to cool to room temperature before serving to the panelists. Each solution was given a random 3-digit code and was served to the panelists in random order. Panelists dispensed 1 mL of each solution into their mouths from a pipette. The panelists were then asked to rate the "sweetness intensity" and "bitterness intensity" of the solutions and mark their responses on an un-anchored, 15 cm line ballot. The length of the line directly corresponded to the scale values (1-15) on which the participants were trained.

In order to prepare the panelists' palates, a control solution of commercial rebiana (300 ppm) was the first sample each panelist tasted during a sitting. In between testing samples, the panelists cleansed their palates with water and apple slices. The panelists also waited 5 minutes between each sample. The panelists' responses were measured, compiled, and averaged for each sample.

**Table 2: Sweet and Bitter Response of the Pure Glycosides**

| **Reb A (ppm)** | **Reb B (ppm)** | **Reb D (ppm)** | **Sweetness** | **Bitterness** |
|---|---|---|---|---|
| 0 | 0 | 126 | 3.5 | 4.3 |
| 0 | 0 | 251 | 6.9 | 5.3 |
| 0 | 0 | 377 | 8.1 | 6.3 |
| 0 | 0 | 503 | 8.6 | 6.1 |
| 0 | 0 | 629 | 9.2 | 6.7 |
| 0 | 0 | 880 | 9.6 | 6.5 |
| 0 | 57 | 0 | 1.8 | 3.9 |
| 0 | 114 | 0 | 2.7 | 4.2 |
| 0 | 171 | 0 | 3.6 | 4.3 |
| 0 | 286 | 0 | 5.8 | 5.0 |
| 0 | 343 | 0 | 6.5 | 5.1 |
| 0 | 400 | 0 | 7.6 | 5.8 |
| 114 | 0 | 0 | 3.1 | 4.2 |
| 229 | 0 | 0 | 5.7 | 5.3 |
| 343 | 0 | 0 | 7.4 | 6.6 |
| 457 | 0 | 0 | 8.4 | 7.0 |
| 571 | 0 | 0 | 9.2 | 8.4 |
| 800 | 0 | 0 | 10.1 | 9.0 |

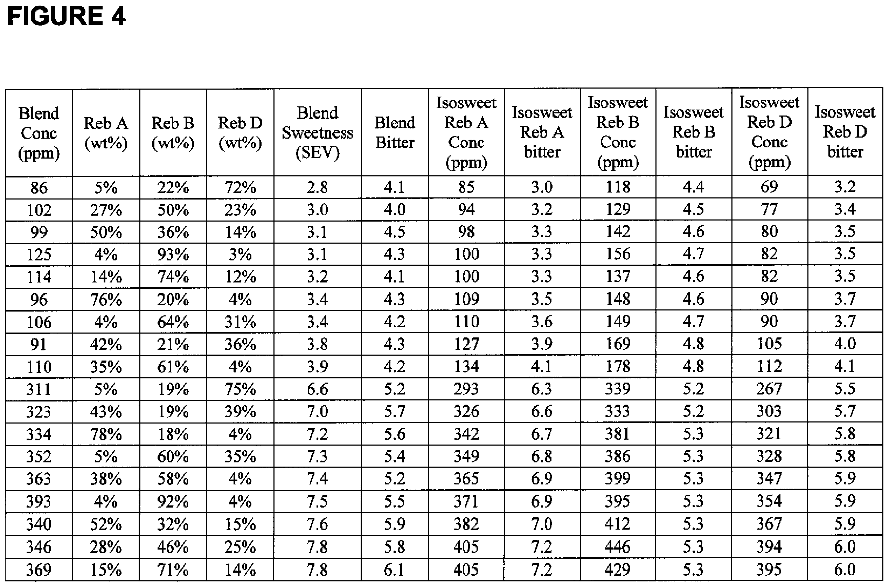

Table 2 describes the sweet and bitter responses of rebaudioside A, rebaudioside B, and rebaudioside D in pure form. The sweet and bitter responses of binary blends are shown in figures 1-3 (REB-BD blends, REB-AD blends, and REB-AB blends respectively). Figure 4 shows the results for ternary blends (REB-ABD blends). As described above, the samples were tasted by the panelists in random order. The results are being presented in table 2 and figures 1-4 as a matter of convenience to more easily display and describe the results.

The figures show the concentration of the blend tested (ppm) as well as the ratio of one glycoside to another in the blend as a percentage. Each blend's sweetness and bitterness was measured by the trained panel. The blend's sweetness is measured as SEV.

Each blend was then compared to an isosweet concentration of the pure glycosides. This value represents the concentration of the pure glycoside needed to achieve the SEV value measured for the blend. Thus, if the value is greater than that of the blend, then a larger concentration of the pure glycoside would be needed to achieve the same sweetness as achieved by the blend (at the lower dosage). The tables also include an isosweet bitterness value for each pure glycoside. This value represents the intensity of bitterness measured for that concentration of pure glycoside. The concentration of the isosweet solution of rebaudioside A, rebaudioside B, or rebaudioside D and the bitterness of the isosweet solutions were calculated by a fit of the pure component sensory response (table 2) to standard psycho-sensory models.

Figure 1 represents data obtained for REB-BD blends. The 3 highest SEV values show a surprising sweetness synergy between rebaudoside B and rebaudioside D at these higher SEV levels. The same sweetness intensity was achieved in these 3 samples with a lower concentration of glycosides in the blend than with either pure rebaudioside B or pure rebaudioside D.

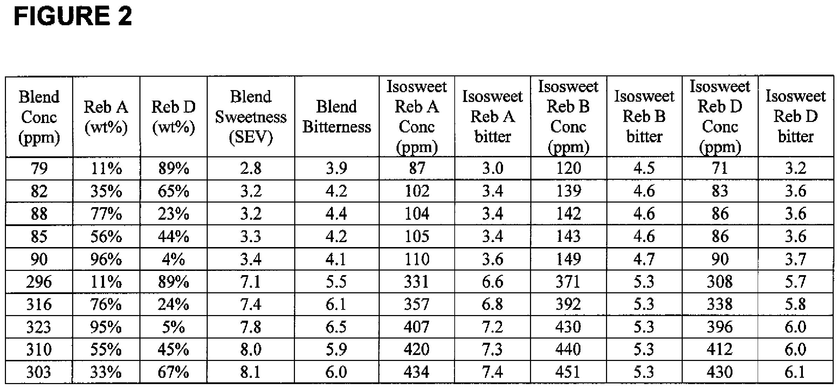

Figure 2 represents data obtained for REB-AD blends. Surprisingly, Certain intermediate ratios of rebaudioside A and rebaudioside D showed sweetness synergy across all SEV levels. Specifically, 33%/67%, 35%/65%, 55%/45%, and 56%/44% rebaudioside A/rebaudioside D blends all showed higher effective sweetening ability than either pure component rebaudioside A or rebaudioside D. Interestingly, at the lower SEV any adjustment outside of these narrow ranges did not yield these benefits.

Blends with the five highest SEV values all showed higher effective sweetening ability than either pure rebaudioside A or rebaudioside D. More surprising was the magnitude of improvement for the two highest, and especially the two highest SEV values. At these highest SEV values, the concentration of pure component rebaudioside A or rebaudioside D needed to reach the blend sweetness was significantly greater. Utilization of these blends could significantly reduce the amount of glycoside needed to achieve a particular sweetness.

Also very unexpected was the improvement in bitterness for the two highest SEV values. At 55%/45% and 33%/67% rebaudioside A/rebaudioside D at SEV of 8.0 and 8.1 respectively, a bitterness reduction was discovered. Thus, not only could significantly less glycoside be used, the glycoside blends would also be less bitter than their pure component counterparts.

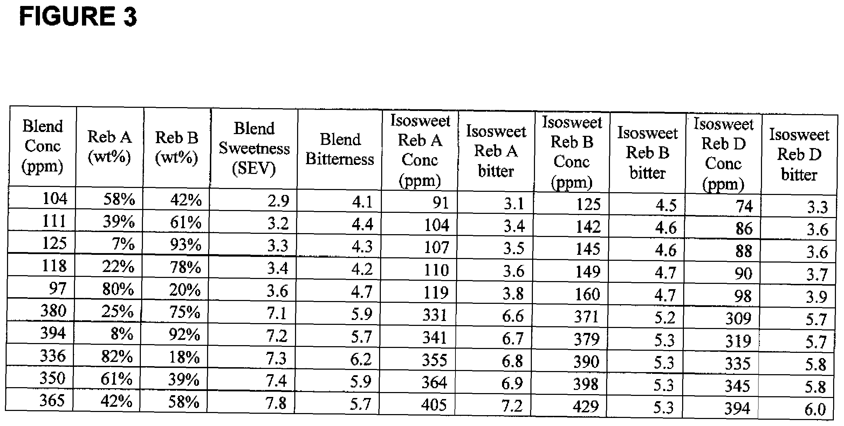

Figure 3 represents the data obtained for REB-AB blends. Particular ratios of rebaudioside A to rebaudioside B at higher SEV levels show higher effective sweetening ability than either pure rebaudioside A or rebaudioside B. Specifically, 82%/18%, 61%/39%, and 42%/58% rebaudioside A/rebaudiosde B all showed higher effective sweetening ability than either pure component rebaudioside A or rebaudioside B. Surprisingly, at similar high SEV levels, blends with less rebaudioside A and more rebaudioside B did not show the same beneficial effect.

Figure 4 shows data obtained for REB-ABD blends. Particular ratios of the three glycosides at higher SEV levels show higher effective sweetening ability than either pure rebaudioside A, rebaudioside B, or rebaudioside D. Specifically, 52%/32%/15%, 28%/46%/25%, and 15%/71%/14% rebaudioside A/rebaudioside B/rebaudioside D blends all showed higher effective sweetening ability than pure component rebaudioside A, rebaudioside B, and rebaudioside D. Surprisingly, blends at similar SEV with low levels of rebaudioside A or D (less than 10%) or lower levels of rebaudioside B (less than 25%) did not show such benefits.

A high intensity sweetener may be chosen from sucralose, aspartame, saccharin, acesulfame K, alitame, thaumatin, dihydrochalcones, neotame, cyclamates, mogroside, glycyrrhizin, phyllodulcin, monellin, mabinlin, brazzein, circulin, pentadin, and combinations thereof. A flavoring may be chosen from a cola flavor, a citrus flavor, a root beer flavor, and combinations thereof. A sweetness enhancer may be chosen from curculin, miraculin, cynarin, chlorogenic acid, caffeic acid, strogins, arabinogalactan, maltol, dihyroxybenzoic acids, and combinations thereof.

Other ingredients such as food starch, flours, protein isolates, protein concentrates, food fats and oils (such as cocoa butter), food extracts (such as malt extract), and juice concentrates may also be included in the sweetener compositions.

In some particular embodiments, the sweetener composition comprising a glycoside blend can also include a lower glycemic carbohydrate. In certain preferred embodiments, the lower glycemic carbohydrate is erythritol or another polyol. In especially preferred embodiments, the sweetener composition includes a particular glycoside blend and erythritol.

In other particular embodiments, the sweetener composition comprising a glycoside blend can also include a fiber. In certain preferred embodiments the fiber is polydextrose, resistant maltodextrin, or inulin.

### Food and Beverage Compositions

The sweetener compositions of the present inventions can also be incorporated into food and beverage compositions. Thus, the present invention also contemplates food compositions and beverage compositions which include the sweetener compositions of the present invention.

### Methods of Producing Sweetener Compositions

The present invention also contemplates methods for producing the sweetener compositions. Typical conventional *Stevia* based sweeteners include a glycoside blend which consists primarily of rebaudioside A (for example greater than 95% rebaudioside A, or greater than 97% rebaudioside A).

The present invention contemplates adding rebaudioside B to such conventional sweeteners. In some embodiments, rebaudioside B could be added to such sweeteners to achieve the desired rebaudioside A to rebaudioside B glycoside blend ratio. In yet other embodiments of the disclosure, rebaudioside B and rebaudioside D could be added to such sweeteners to achieve the desired rebaudioside A to rebaudioside B to rebaudioside D glycoside blend ratio.

The present invention also contemplates controlled conversion between one glycoside and another glycoside to achieve the glycoside blends of the present invention. Thus, in one embodiment, a substantially pure rebaudioside A composition can be converted to particular REB-AB blend, REB-BD blend, or REB-ABD blend at the claimed ratios.

### REFERENCE EXAMPLE

### Example 1: Sensory Testing of Various Glycoside Blends

A 20 person sensory panel was trained to scale sweetness and bitterness. Reference tasting standards were prepared by dissolving respective standard material (sucrose for sweetness and caffeine for bitterness) into reverse osmosis water according to the scale values shown in Table 1 below.

**Table 1: Reference Tasting Standards**

| | Concentration (g/kg) | |
|---|---|---|
| | Sucrose | Caffeine |
| Scale | *(Sweetness)* | *(Bitterness)* |
| 1 | 10 | 0.107 |
| 2 | 20 | 0.153 |
| 3 | 30 | 0.200 |
| 4 | 40 | 0.246 |
| 5 | 50 | 0.293 |
| 6 | 60 | 0.340 |
| 7 | 70 | 0.386 |
| 8 | 80 | 0.433 |
| 9 | 90 | 0.479 |
| 10 | 100 | 0.526 |
| 11 | 110 | 0.572 |
| 12 | 120 | 0.619 |
| 13 | 130 | 0.666 |
| 14 | 140 | 0.712 |
| 15 | 150 | 0.759 |

Pure rebaudioside A, rebaudioside B, and rebaudioside D were obtained. Rebaudioside A (99% purity) was obtained from ChromaDex^{®}. Rebaudioside B (97.3% purity) was obtained from Cargill, Incorporated. Rebaudioside D (92.5% purity) was obtained from a commercial source.

The trained sensory panel evaluated pure and blended solutions of rebaudioside A, rebaudioside B, and rebaudioside D at ratios and concentrations shown in the tables and in figures 1-4. Solutions were made in Evian^{®} water. All solutions were heated to 47°C for 10 minutes to ensure that all the glycoside material was completely dissolved. The solutions were allowed to cool to room temperature before serving to the panelists. Each solution was given a random 3-digit code and was served to the panelists in random order. Panelists dispensed 1 mL of each solution into their mouths from a pipette. The panelists were then asked to rate the "sweetness intensity" and "bitterness intensity" of the solutions and mark their responses on an un-anchored, 15 cm line ballot. The length of the line directly corresponded to the scale values (1-15) on which the participants were trained.

In order to prepare the panelists' palates, a control solution of commercial rebiana (300 ppm) was the first sample each panelist tasted during a sitting. In between testing samples, the panelists cleansed their palates with water and apple slices. The panelists also waited 5 minutes between each sample. The panelists' responses were measured, compiled, and averaged for each sample.

**Table 2: Sweet and Bitter Response of the Pure Glycosides**

| **Reb A (ppm)** | **Reb B (ppm)** | **Reb D (ppm)** | **Sweetness** | **Bitterness** |
|---|---|---|---|---|
| 0 | 0 | 126 | 3.5 | 4.3 |
| 0 | 0 | 251 | 6.9 | 5.3 |
| 0 | 0 | 377 | 8.1 | 6.3 |
| 0 | 0 | 503 | 8.6 | 6.1 |
| 0 | 0 | 629 | 9.2 | 6.7 |
| 0 | 0 | 880 | 9.6 | 6.5 |
| 0 | 57 | 0 | 1.8 | 3.9 |
| 0 | 114 | 0 | 2.7 | 4.2 |
| 0 | 171 | 0 | 3.6 | 4.3 |
| 0 | 286 | 0 | 5.8 | 5.0 |
| 0 | 343 | 0 | 6.5 | 5.1 |
| 0 | 400 | 0 | 7.6 | 5.8 |
| 114 | 0 | 0 | 3.1 | 4.2 |
| 229 | 0 | 0 | 5.7 | 5.3 |
| 343 | 0 | 0 | 7.4 | 6.6 |
| 457 | 0 | 0 | 8.4 | 7.0 |
| 571 | 0 | 0 | 9.2 | 8.4 |
| 800 | 0 | 0 | 10.1 | 9.0 |

Table 2 describes the sweet and bitter responses of rebaudioside A, rebaudioside B, and rebaudioside D in pure form. The sweet and bitter responses of binary blends are shown in figures 1-3 (REB-BD blends, REB-AD blends, and REB-AB blends respectively). Figure 4 shows the results for ternary blends (REB-ABD blends). As described above, the samples were tasted by the panelists in random order. The results are being presented in table 2 and figures 1-4 as a matter of convenience to more easily display and describe the results.

The figures show the concentration of the blend tested (ppm) as well as the ratio of one glycoside to another in the blend as a percentage. Each blend's sweetness and bitterness was measured by the trained panel. The blend's sweetness is measured as SEV.

Each blend was then compared to an isosweet concentration of the pure glycosides. This value represents the concentration of the pure glycoside needed to achieve the SEV value measured for the blend. Thus, if the value is greater than that of the blend, then a larger concentration of the pure glycoside would be needed to achieve the same sweetness as achieved by the blend (at the lower dosage). The tables also include an isosweet bitterness value for each pure glycoside. This value represents the intensity of bitterness measured for that concentration of pure glycoside. The concentration of the isosweet solution of rebaudioside A, rebaudioside B, or rebaudioside D and the bitterness of the isosweet solutions were calculated by a fit of the pure component sensory response (table 2) to standard psycho-sensory models.

Figure 1 represents data obtained for REB-BD blends. The 3 highest SEV values show a surprising sweetness synergy between rebaudoside B and rebaudioside D at these higher SEV levels. The same sweetness intensity was achieved in these 3 samples with a lower concentration of glycosides in the blend than with either pure rebaudioside B or pure rebaudioside D.

Figure 2 represents data obtained for REB-AD blends. Surprisingly, Certain intermediate ratios of rebaudioside A and rebaudioside D showed sweetness synergy across all SEV levels. Specifically, 33%/67%, 35%/65%, 55%/45%, and 56%/44% rebaudioside A/rebaudioside D blends all showed higher effective sweetening ability than either pure component rebaudioside A or rebaudioside D. Interestingly, at the lower SEV any adjustment outside of these narrow ranges did not yield these benefits.

Blends with the five highest SEV values all showed higher effective sweetening ability than either pure rebaudioside A or rebaudioside D. More surprising was the magnitude of improvement for the two highest, and especially the two highest SEV values. At these highest SEV values, the concentration of pure component rebaudioside A or rebaudioside D needed to reach the blend sweetness was significantly greater. Utilization of these blends could significantly reduce the amount of glycoside needed to achieve a particular sweetness.

Also very unexpected was the improvement in bitterness for the two highest SEV values. At 55%/45% and 33%/67% rebaudioside A/rebaudioside D at SEV of 8.0 and 8.1 respectively, a bitterness reduction was discovered. Thus, not only could significantly less glycoside be used, the glycoside blends would also be less bitter than their pure component counterparts.

Figure 3 represents the data obtained for REB-AB blends. Particular ratios of rebaudioside A to rebaudioside B at higher SEV levels show higher effective sweetening ability than either pure rebaudioside A or rebaudioside B. Specifically, 82%/18%, 61%/39%, and 42%/58% rebaudioside A/rebaudiosde B all showed higher effective sweetening ability than either pure component rebaudioside A or rebaudioside B. Surprisingly, at similar high SEV levels, blends with less rebaudioside A and more rebaudioside B did not show the same beneficial effect.

Figure 4 shows data obtained for REB-ABD blends. Particular ratios of the three glycosides at higher SEV levels show higher effective sweetening ability than either pure rebaudioside A, rebaudioside B, or rebaudioside D. Specifically, 52%/32%/15%, 28%/46%/25%, and 15%/71%/14% rebaudioside A/rebaudioside B/rebaudioside D blends all showed higher effective sweetening ability than pure component rebaudioside A, rebaudioside B, and rebaudioside D. Surprisingly, blends at similar SEV with low levels of rebaudioside A or D (less than 10%) or lower levels of rebaudioside B (less than 25%) did not show such benefits.

## Claims

1. A sweetener composition comprising a glycoside blend, wherein
the glycoside blend comprises from 40% to 85% rebaudioside A and from 15% to 60% rebaudioside B (of the total rebaudioside A and rebaudioside B in the glycoside blend), and the glycoside blend provides an SEV of greater than 7.2, in the sweetener composition, and rebaudioside A and rebaudioside B comprise at least 60% of the glycoside blend,
wherein the composition further comprises a bulking agent.

2. The sweetener composition of claim 1, wherein rebaudioside A and rebaudioside B comprise at least 90% of the glycoside blend.

3. The sweetener composition of claim 1, wherein the sweetener composition further comprises a low glycemic carbohydrate.

4. The sweetener composition of claim 1, where the sweetener composition further comprises erythritol.

5. The sweetener composition of claim 1, where the sweetener composition further comprises inulin.

6. A food composition comprising the sweetener composition of any of claims 1-5.

7. A beverage composition comprising the sweetener composition of any of claims 1 -5.

## Patentansprüche

1. Süßstoffzusammensetzung, die eine Glykosidmischung umfasst, wobei die Glykosidmischung von 40 % bis 85 % Rebaudiosid A und von 15 % bis 60 % Rebaudiosid B (des gesamten Rebaudiosid A und Rebaudiosid B in der Glykosidmischung) umfasst, und die Glykosidmischung einen SEV-Wert (Saccharose äquivalenten Wert) größer als 7,2, in der Süßstoffzusammensetzung bereitstellt, und Rebaudiosid A und Rebaudiosid B wenigstens 60 % der Glykosidmischung umfassen, wobei die Zusammensetzung ferner einen Füllstoff umfasst.

2. Süßstoffzusammensetzung nach Anspruch 1, wobei das Rebaudiosid A und das Rebaudiosid B wenigstens 90 % der Glykosidmischung umfassen.

3. Süßstoffzusammensetzung nach Anspruch 1, wobei die Süßstoffzusammensetzung ferner ein niedriges glykämisches Kohlehydrat umfasst.

4. Süßstoffzusammensetzung nach Anspruch 1, wobei die Süßstoffzusammensetzung ferner Erythrit umfasst.

5. Süßstoffzusammensetzung nach Anspruch 1, wobei die Süßstoffzusammensetzung ferner Inulin umfasst.

6. Nahrungsmittelzusammensetzung, welche die Süßstoffzusammensetzung nach irgendeinem der Ansprüche 1-5 umfasst.

7. Getränkezusammensetzung, welche die Süßstoffzusammensetzung nach irgendeinem der Ansprüche 1-5 umfasst.

## Revendications

1. Une composition d'édulcorant comprenant un mélange de glycosides, dans laquelle
le mélange de glycosides comprend de 40 % à 85 % de rebaudioside A et de 15 % à 60 % de rebaudioside B (du total de rebaudioside A et de rebaudioside B dans le mélange de glycosides), et le mélange de glycosides présente une valeur SEV supérieure à 7,2, dans la composition d'édulcorant, et le rebaudioside A et le rebaudioside B constituent au moins 60 % du mélange de glycosides, dans laquelle la composition comprend en outre un agent gonflant.

2. La composition d'édulcorant selon la revendication 1, dans laquelle le rebaudioside A et le rebaudioside B constituent au moins 90 % du mélange de glycosides.

3. La composition d'édulcorant selon la revendication 1, dans laquelle la composition d'édulcorant comprend en outre un glucide à faible indice glycémique.

4. La composition d'édulcorant selon la revendication 1, où la composition d'édulcorant comprend en outre de l'érythritol.

5. La composition d'édulcorant selon la revendication 1, où la composition d'édulcorant comprend en outre de l'inuline.

6. Une composition d'aliment comprenant la composition d'édulcorant selon l'une quelconque des revendications 1-5.

7. Une composition de boisson comprenant la composition d'édulcorant selon l'une quelconque des revendications 1 -5.
